# EUROPEAN PATENT APPLICATION

(11) **EP 2 123 664 A1**
(43) Date of publication of application: **25.11.2009**
(21) Application number: 08711135.7
(22) Date of filing: 12.02.2008
(51) Int. Cl.: C07H 15/26, A61K 31/7056, A61P 35/00, A61P 43/00

(54) **NOVEL SUGAR-LINKED CHLORIN DERIVATIVE AND PROCESS FOR PRODUCTION THEREOF**

(30) Priority: 19.02.2007 JP 2007038489
(71) Applicant: Rei Medical Co., Ltd., Nishikyo-ku Kyoto-shi Kyoto 615-8245 (JP)
(72) Inventor: OBATA, Makoto, Nara-shi Nara 630-8301 (JP); YANO, Shigenobu, Nara-shi Nara 631-0033 (JP); HIROHARA, Shiho, Nara-shi Nara 630-8114 (JP)
(74) Representative: De Clercq, Ann G. Y.
(86) International application number: PCT/JP2008/052277
(87) International publication number: WO 2008/102669

(57) **Abstract**

Disclosed is a novel glycoconjugated chlorin derivative which is useful as a substance for photodynamic therapy, is stable, and can act a photosensitizer having high phototoxicity even when used in a small quantity. Also disclosed is a method of producing the derivative. Specifically disclosed are a S-glycosylated chlorin derivative represented by the following general formula (1) and a metal complex thereof. In the general formula (1), X¹ to X²⁰ independently represent a group selected from the group consisting of F-, sugar-S-, sugar-Z-Sand sugar-O-Z-S- and at least one among X¹ to X²⁰ represents a group selected from the group consisting of sugar-S-, sugar-Z-S- and sugar-O-Z-S- (wherein F represents a fluorine atom, Z represents an oxygen atom, Z represents a hydrocarbon group having 1 to 6 carbon atoms); and R⁵ and R⁶ independently represent a hydrogen atom or an organic group or may together form a ring.

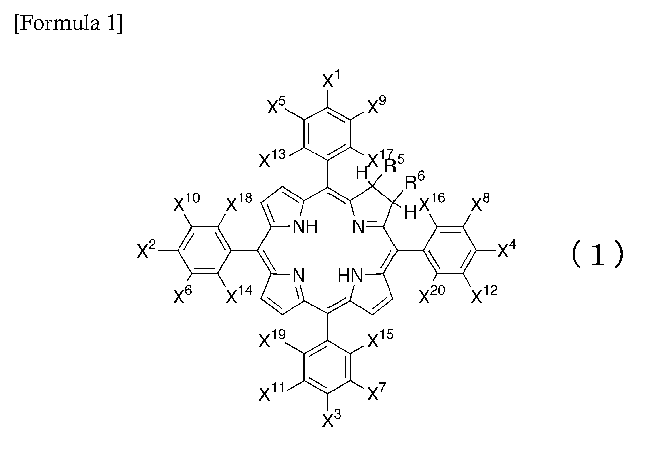

## Description

### TECHNICAL FIELD

The present invention relates to novel glycoconjugated chlorin derivatives with high phototoxicity and a method of producing thereof, as well as pharmaceutical compositions containing the chlorin derivatives.

### BACKGROUND ART

Photodynamic therapy (PDT) is a method of treatment, in which photosensitizer is administered to target diseased tissue and is accumulated in cancer or tumor tissue or diseased area followed by irradiation with light of appropriate wavelength to selectively destroy cancer tissue. PDT utilizes the action of a photosensitizer which is excited by irradiation with light of specific wavelength and damages body tissues. The main mechanism of tumor destruction is thought to be the formation of singlet molecular oxygen from triplet oxygen by direct or indirect transfer of energy which is generated by activated photosensitizer.

For photosensitizers used for PDT, porphyrin derivatives are well known, among which polyhematoporphyrin ether (e.g., Photofrin (trademark)) and sodium talaporphyrin have already been in practical application. However, since hematoporphyrin and its dimer have a maximum absorption wavelength of 630 nm and low molar absorbance coefficient of 3000, the treatment effectiveness of PDT is restricted in superficial cancers with a depth of 5 to 10mm. And side-effects such as photosensitivity reaction also remain a problem. Therefore, a variety of porphyrin derivatives with broader application and less side effects have been studied and proposed.

In general, PDT damages not only cancer or tumor tissue, but also normal tissue. For this reason, it is required to use photosensitizer which can exhibit phototoxicity in the smallest possible quantity. Furthermore, in order to alleviate side-effects, cytotoxicity should be low in the absence of light irradiation. Recently, it has been found that a porphyrin ring with sugar attached can show reduced cytotoxicity in the dark while maintaining strong cytotoxicity when exposed to irradiation of light, and the application of glycoconjugated porphyrin derivatives to PDT drug has been proposed.

For example, the patent reference 1 proposes tetraphenyl bacteriochlorin derivatives and metal complexes thereof, wherein monosaccharides such as glucose or acetylated monosaccharides are bound to the phenyl groups by O-glycosylation. O-glycosylated tetraphenylbacteriochlorin derivatives are obtained by first preparing O-glycosylated tetraphenylporphyrin derivatives with acetylated sugar, which is then reduced with a reducing agent in the presence of an alkali metal carbonate to give tetraphenylbacteriochlorin derivatives, followed by treatment with alkali for deacetylation of the acetylated sugar to make hydroxyl groups of monosaccharide residues be in a free state.

Also, it is disclosed that 5,10,15,20-tetrakis[3-(β-D-glucopyranosyloxy)phenyl]bacteriochlorin, 5,10,15,20-tetrakis[3-(β-D-arabinopyranosyloxy)phenyl]bacteriochlorin, 5,10,15,20-tetrakis[3-(β-D-xylopyranosyloxy)phenyl]bacteriochlorin are prepared and these O-glycosylated bacteriochlorin derivatives have a strong absorption peak near 740 nm, and these bacteriochlorin derivatives show cytotoxicity upon irradiation with light.

In the non-patent reference 1, they report that O-glycosylated chlorin derivatives such as 5,10,15,20-tetrakis[3-(β-D-glucopyranosyloxy)phenyl]chlorin are prepared and the compounds show cytotoxicity by irradiation with light, while no cytotoxicity is found in the dark.

In the patent reference 2, porphyrin derivatives conjugated with monosaccharides such as galactose, xylose and glucose, and with disaccharides such as maltose by S-glycosylation are proposed. Specifically, a halogenated alkoxyphenylporphyrin derivative is reacted with thio sugar acetyl in order to substitute the halogen attached to the alkoxy group with thio sugar acetyl, followed by deacetylation to produce a S-glycosylated alkoxyporphyrin derivative. When a porphyrin ring is attached with a pentafluorophenyl group in place of the alkoxyphenyl group, the pentafluorophenylporphyrin is reacted with thio sugar acetyl to form a S-glycosylated tetrafluorophenylporphyrin.

And it is disclosed that these glycoconjugated porphyrin derivatives show cytotoxicity by irradiation with light, and the derivatives have stronger phototoxic activity than non glycosylated analogues of porphyrin derivatives.
In the patent reference 2, it is described that a metal complex can be made, if desired, by the treatment with an appropriate ion such as Mg, Zn, Sn and the like, however, its preparation example does not exist.

The non patent reference 2 also reports about S-glycosylated tetraphenylporphyrin. In the reference, the tetra(pentafluorophenyl)porphyrin (TPPF₂₀) is synthesized, and this TPPF₂₀ is reacted with thioacetate derivatives of sugar to produce S-glycosylated tetra(pentafluorophenyl)porphyrins, such as 5, 10, 15, 20-tetrakis(4, 1'-thio-glucosyl-2, 3, 5, 6-tetrafluorophenyl)porphyrin, and after these porphyrin derivatives are contacted with some cells, cell death can be observed with irradiation of light, and moreover the effect of phototoxicity differs according to the variety of sugars.

Patent reference 1: WO2002-020536
Patent reference 2: JP2005-500335A
Non patent reference 1: Shiho Hirohara et.al., "Sugar-dependent aggregation of glycoconjugated chlorins and its effect on photocytotoxicity in HeLa cells", Journal of Photochemistry and Photobiology B: Biology 84, 2006, 56-63
Non patent reference 2: Xin Chen et.al., "Efficient synthesis and photodynamic activity of porphyrin-saccharide conjugates: targeting and incapacitating cancer cells", Biochemistry 2004, 43, 10918-10929

### DISCLOSURE OF THE INVENTION

### PROBLEMS TO BE SOLVED BY THE INVENTION

As shown in the above, glycoconjugated porphyrin, chlorin derivatives have also been developed and studied, however, further improvement has to be made in phototoxicity, safety, and productivity. The present invention therefore provides novel glycoconjugated chlorin derivatives which are stable in the dark and show high phototoxicity in small quantity and for which productivity is high, and methods of producing the same.

### MEANS OF SOLVING THE PROBLEMS

The present inventors studied various glycoconjugated porphyrin derivatives and chlorin derivatives, and then found novel glycoconjugated chlorin derivatives as a useful photosensitizer for photodynamic therapy, having stability in the dark and high phototoxicity in a small amount, and a method of producing the chlorin derivatives.

The present invention provides a S-glycocylated chlorin derivative represented by the following general formula (1) and a metal complex of the S-glycosylated chlorin derivative represented by the following general formula (2), as a novel glycoconjugated chlorin derivative.

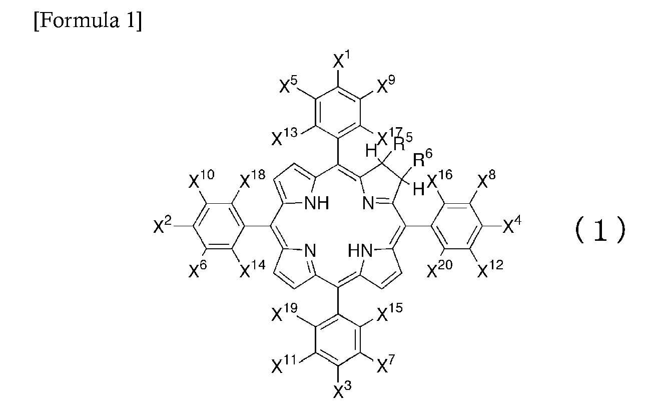

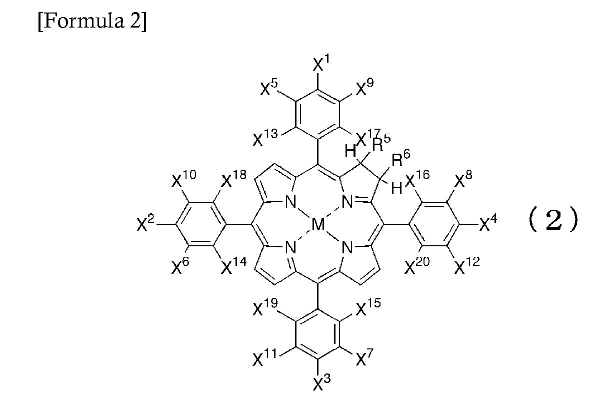

In the above formulae, X¹ to X²⁰ independently one another represent a group selected from the group consisting of F-, sugar-S-, sugar-Z-S- and sugar-O-Z-S-, and at least one among X¹ to X²⁰ represents a group selected from the group consisting of sugar-S-, sugar-Z-S-, and sugar-O-Z-S- (where F represents a fluorine atom, S represents a sulfur atom, O represents an oxygen atom, and Z represents a hydrocarbon group having 1 to 6 carbon atoms); and R⁵ and R⁶ independently represent a hydrogen atom or an organic group or may together form a ring.

Preferably, as shown in the formula (1)' or (2)', at least one of X¹, X², X³, and X⁴, which are on p-position of the chlorin ring, is a group selected from the group consisting of sugar-S-, sugar-Z-S-, and sugar-O-Z-S-, and the atom on o- or m- position of the chlorin ring is fluorine. More preferably, all of X¹, X², X³, and X⁴ are sugar-S-.

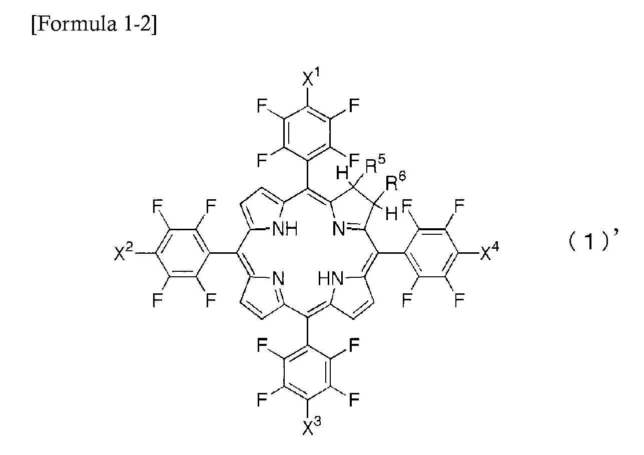

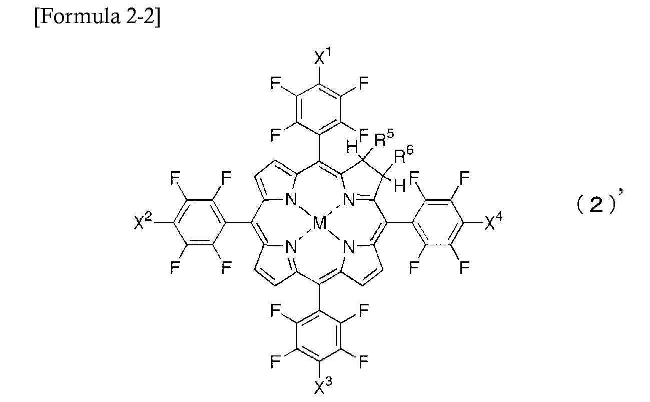

The sugar selected from the group consisting of monosaccharide, disaccharide, and polysaccharide is preferred, pentose or hexose represented by the following formulae is more preferred, glucose is further more preferred. The sugar of D-form is preferable.

.

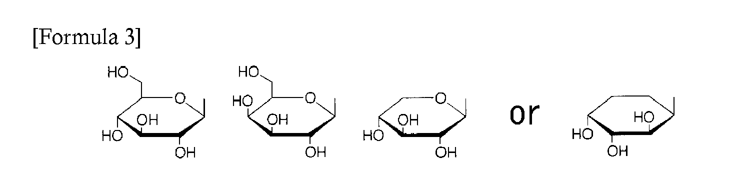

The S-glycosylated chlorin derivatives of the present invention are used for destructing a target by a method comprising contacting in vitro or in vivo the chlorin derivative with a target selected from the group consisting of virus, bacteria, or infected cell thereby, tumorous cell, and tumorous tissues; and irradiating a light of a wavelength absorbable to the chlorin derivative. Accordingly, the present invention also provides a medicine, particularly, a medicine useful for photodynamic therapy for tumor, solid cancer or skin disease, and diagnostic reagent, which contain the chlorin derivative or the metal complex thereof.

In another aspect, the present invention provides a method of producing the S-glycosylated chlorin derivative or a metal complex thereof.
The producing method of the chlorin derivative of the general formula (1) comprises a step of preparing tetrakis(pentafluorophenyl)chlorin via a process of subjecting the A ring of tetraphenylporohyrin to a reduction reaction or an addition reaction to afford tetraphenylchrolin; and a step of S-glycosylation by reacting the tetrakis(pentafluorophenyl)chlorin with a thiolester of a sugar or sugar derivative.

Preferably, the tetraphenyl porphyrin having fluorated phenyls beforehand, i.e. tetrakis(pentafluorophenyl)porphyrin is used. Accordingly, a preferable producing method comprises a step of subjecting the A ring of tetrakis(pentafluorophenyl)porphyrin to a reduction reaction or an addition reaction to afford tetrakis(pentafluorophenyl)chrolin; a step of separating and removing tetrakis(pentafluorophenyl)porphyrin from the resulting reaction mixture to obtain the tetrakis(pentafluorophenyl)chlorin; and a step of S-glycosylation by reacting the tetrakis(pentafluorophenyl)chlorin with a thiolester of a sugar or sugar derivative.

A method of producing a metal complex of the chlorin derivative represented by the above-mentioned general formula (2) comprises a step of preparing a metal complex of tetrakis(pentafluorophenyl)chlorin via a process of subjecting the A ring of tetraphenylporphyrin to a reduction reaction or an addition reaction to afford tetraphenylchrolin, and a process of reacting a tetraphenylporphyrin or tetraphenylchrolin with a salt of the metal; and a step of S-glycosylation by reacting the metal complex of tetrakis(pentafluorophenyl)chlorin with a thiolester of a sugar or sugar derivative.

A preferable producing method of the metal complex of the chlorin derivative represented by the general formula (2) comprises a step of preparing a metal complex of tetrakis(pentafluorophenyl)porphyrin by reacting a tetrakis(pentafulorophenyl)porphyrin with a salt of the metal; a step of subjecting the A ring of porphyrin of the metal complex of tetrakis(pentafluorophenyl)porphyrin to a reduction reaction or an addition reaction to afford a metal complex of tetrakis(pentafluorophenyl)chrolin; a step of separating and removing tetrakis(pentafluorophenyl)chlorin from the resulting reaction mixture to obtain a metal complex of tetrakis(pentafluorophenyl)chlorin; and a step of S-glycosylation by reacting the metal complex of tetrakis(pentafluorophenyl)chlorin with a thiolester of a sugar or sugar derivative.

The glycoconjugated chlorin derivatives of the present invention are no toxicity in the dark, but can show strong cytotoxicity by irradiation with light.
The producing method of the present invention can produce the glycoconjugated chlorin derivatives of the invention with high productivity, and also produce a metal complex of the chlorin derivative having high melting point.

### BRIEF DESCRIPTION OF DRAWINGS

Fig. 1 illustrates an embodiment of the producing method of glycoconjugated chlorin derivatives of the invention.
Fig. 2 illustrates an embodiment of the producing method of glycoconjugated chlorin derivatives of the invention.
Fig. 3 is a graph showing the result of phototoxicity test at a drug concentration of 0.5 µM.
Fig. 4 is a graph showing the result of phototoxicity test (No. 6, 7, 8, 9, 14, 15) at a drug concentration of 0.09 µM.
Fig. 5 is a graph showing the result of phototoxicity test (No. 8, 16) at a drug concentration of 0.09 µM.
Fig. 6 is a graph showing the result of cytotoxicity test in the dark at a drug concentration of 0.5 µM.
Fig. 7 shows the ESI-MS spectrum of Example 4 (H₂TFPC-SCH₂CH₂OAcGlc).
Fig. 8 shows the simulation result of the ESI-MS spectrum of Example 4 (H₂TFPC-SCH₂CH₂OAcGlc).
Fig. 9 shows the ESI-MS spectrum of Example 5 (H₂TFPC'-SAcGlc).
Fig. 10 shows the simulation result of ESI-MS spectrum of Example 5 (H₂TFPC'-SAcGlc).

### BEST MODE FOR CARRYING OUT THE INVENTION

### [S-glycosylated chlorin derivatives and its producing method]

The present invention provides S-glycosylated chlorin derivatives of the following general formula (1) as novel photosensitizers:

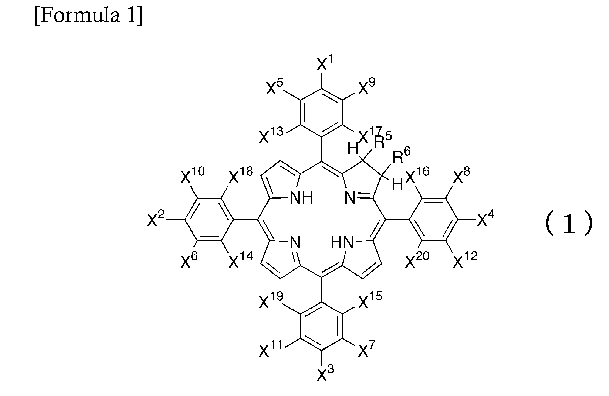

In the formula (1), X¹ to X²⁰ are groups each independently selected from the group consisting of F-, sugar-S-, sugar-Z-S-, and sugar-O-Z-S-, and at least one of X¹ to X²⁰ is a group selected from the group consisting of sugar-S-, sugar-Z-S- and sugar-O-Z-S-.

F is a fluorine atom, S is a sulfur atom, O is an oxygen atom, and Z is a hydrocarbon group having 1 to 6 carbon atoms, such as methylene, ethylene, propylene, α-butylene, β-butylene, cyclohexylen, o-phenylene, m-phenylene, or p-phenylene.

For the glycosylated chlorin derivatives of the invention, at least one of the four phenyl groups bound to the chlorin should be S-glycosylated, either o-, m- or p-position for the chlorin ring can be S-glycosylated. And each phenyl group may be S-glycosylated in more than one position.

Preferably, as shown in the formula (1)' below, at least one of X¹, X², X³, and X⁴ in the p-position for the chlorin ring is a group selected from the group consisting of sugar-S-, sugar-Z-S- and sugar-O-Z-S-, and fluorine atoms are attached to the o- and m-positions in each phenyl group, and more preferably, all of X¹, X², X³, and X⁴ in the p-position are glycosylated. A sugar can be linked directly to a thio group, or a linking group of -Z-, -O-Z- can exist between S atom and a sugar. Preferably, a sugar is directly linked to a thio group.

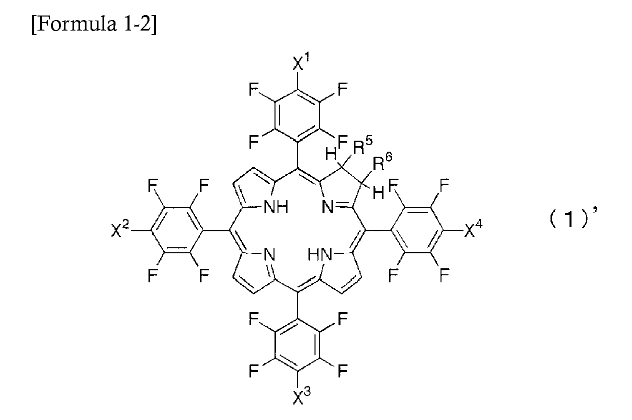

The sugar to be linked includes monosaccharides such as pentose and hexose represented by the formula below; disaccharides such as sucrose, maltose and lactose; polysaccharides such as starch, amylose and glycogen. Monosaccharides are preferred, and pentose or hexose is more preferred. The monosaccharides can be D-form or L-form, however, D-form is preferably used.

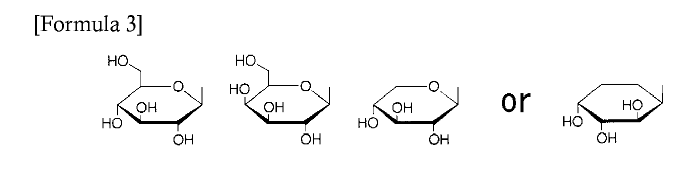

Pentose includes, arabinose, xylose and rhamnose. Specifically, glucose, galactose, mannose, allose, altrose, gulose, idose, tallose are included in hexose, among which glucose is most preferably used. It is because when glucose is linked, the derivative shows high phototoxicity.

In the formula (1) and (1)', when more than one group of X¹ to X²⁰ are substituted with sugar-S-, sugar-Z-S-, and/or sugar-O-Z-S- (hereinafter referred to as "S-glycosylated group" unless otherwise distinguished), the kinds of sugar to be linked may be the same or different. Also, different kinds of sugars like monosaccharides and polysaccharides can be linked. Preferably, all sugars are the same in kind, more preferably, all the sugars contained in the S-glycosylated groups substituted are glucose.

In the above formula (1) and (1)', R⁵ and R⁶ represent an hydrogen atom or, independently of each other, an organic group.
The said organic group includes lower alkyl or Q-(CH₂)ₖ-. Q is lower alkyl, aryl, amino, lower alkylamino, imino, lower alkylimino, or cycloalkyl group. The term "lower alkyl" refers to a linear or branched alkyl comprising 1 to 5 carbon atoms, including methyl, ethyl, n-propyl, isopropyl, n-butyl, sec-butyl, tert-butyl and pentyl. k is an integer of 0 to 3.
R⁵ and R⁶ may be taken together to form a ring, for example, R⁵ and R⁶ may constitute an imino group or a tertiary amine which binds to the 2-position and the 3-position of the A ring in the 1-24 numbering system for porphyrin ring.

Preferred examples of the above-described S-glycosylated chlorin derivatives include 5, 10, 15, 20-tetrakis[4-(β-D-glucopyranosylthio)-2, 3, 5, 6-tetrafluorophenyl]chlorin, 5, 10, 15, 20-tetrakis[4-(β-D-glucopyranosylthio)-2, 3, 5, 6-tetrafluorophenyl]-2, 3-[methano(N-methyl)iminomethano]chlorin, and so on.

S-glycosylated chlorin derivatives having the constitution described above can exhibit strong cytotoxicity with irradiation of light, while they do not show cytotoxicity in the dark. Especially, they exhibit stronger cytotoxicity than O-glycosylated chlorin derivatives. And they have a large absorption at longer wavelengh (maximum absorption wavelength: 650 nm) compared with porphyrin derivatives, and sugar linkage makes the compounds water soluble, which leads higher cell affinity and permeability.

Next, methods to produce the S-glycosylated chlorin derivatives of the invention are described. The methods of producing the S-glycosylated chlorin derivatives of the invention comprise a process to obtain tetrakis(pentafluorophenyl)chlorin via a process of subjecting the A ring of tetraphenylporphyrin to a reduction reaction or an addition reaction to afford tetraphenylchlorin; and a process of S-glycosylation by reacting said tetrakis(pentafluorophenyl)chlorin with thiolester of sugar or sugar derivatives.

Aromatic rings attached to the chlorin can be fluorinated after the conversion of porphyrin to chlorin, whereas a porphyrin attached with previously fluorinated aromatic rings, i.e. tetrakis(pentafluorophenyl)porphyrin is preferably used for the conversion to chlorin. Thus, the preferred methods of preparing the S-glycosylated chlorin derivatives of the invention comprise a process of subjecting the A ring of tetrakis(pentafluorophenyl)porphyrin to a reduction reaction or an addition reaction to give tetrakis(pentafluorophenyl)chlorin; a process of separating and removing the tetrakis(pentafluorophenyl)porphyrin from the resulting reaction mixture to afford tetrakis(pentafluorophenyl)chlorin; and a process of S-glycosylation by reacting said tetrakis(pentafluorophenyl)chlorin with thiolester of sugar or sugar derivatives.

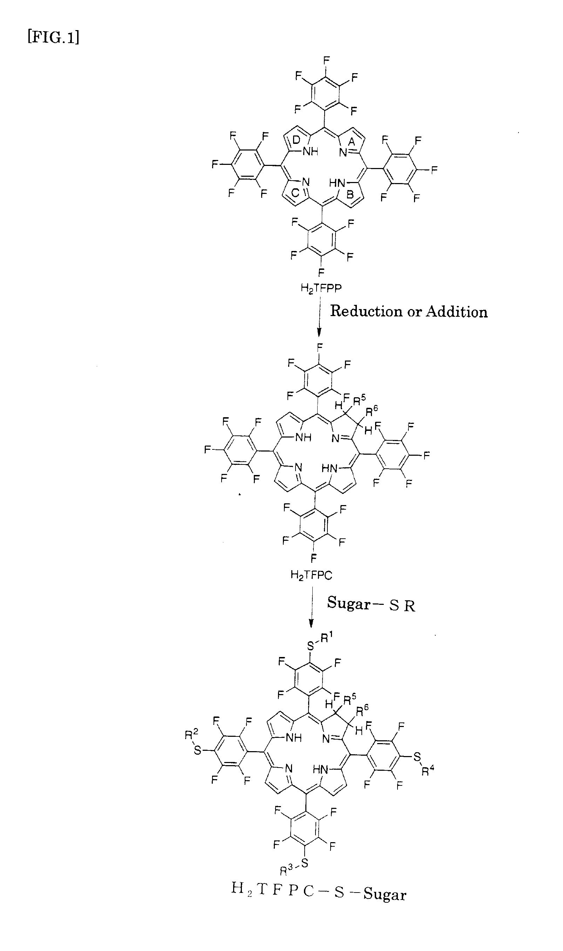

The methods of producing of the invention by using tetrakis(pentafluorophenyl)porphyrins are described in detail with reference to Fig. 1.

First, 5, 10, 15, 20-tetrakis(pentafluorophenyl)porphyrin (abbr. H₂TFPP) is used as a starting material, 2- and 3-position (of the pyrrol A ring) in the 1-24 numbering system for porphyrin ring are subjected to a reduction reaction or an addition reaction with appropriate compounds to give a chlorin ring. Reduction can be conducted with a reducing agent, e.g., hydrazone, semicarbazone, p-toluenesulfonylhydrazide in the most suitable amount for each group in the presence of about three equivalents of an alkali metal carbonate. For the addition reaction, 2- and 3-position of the pyrrol A ring may be subjected to a cycloaddition reaction with a combination of 1, 3-dihydrobenzothiophen 2, 2-dioxide, N-methylglycine and paraformaldehyde, etc. to make the double bond of the A ring to a single bond. Alternatively, halogenated benzyl such as benzyl bromide, or halogenated alkyl, etc. may be used for an addition reaction for the pyrrol A ring in the presence of a chain transfer agent like tributyltin hydride to make the double bond of the A ring to a single bond.

After the reaction, H₂TFPP is separated and removed from the reaction mixture. The separation and removal method can be performed by recrystallization, silica gel chromatography, gel permeation chromatography and so on.
Following the separation and removal of the porphyrin derivative, the resulting tetrakis(pentafluorophenyl)chlorin derivative is reacted with thiolester of sugar (sugar-SR) to link the sugar sulfide to the tetrafluorophenyl group to give a S-glycosylated tetrafluorophenyl chlorin derivative (H₂TFPC-S-sugar). H₂TFPC-S-sugar in Fig. 1 shows the compound bearing sugars in the p-positions of all the 4 phenyl groups. In the H₂TFPC-S-sugar, R¹, R², R³ and R⁴ are the sugar residues derived from the sugar-SR.

The amount of the thiolester of sugar (sugar-SR) to be added may be decided according to the number of the phenyl groups for which sugars are to be linked among 4 pentafluorophenyl groups linked to a chlorin ring. For example, when an excessive amount of the tiolester of sugar is added, more than one sugar is supposed to be linked to one or more phenyl groups of the 4 phenyl groups attached to a chlorin ring, and when an inadequate amount of sugar thiolester is added, a glycoconjugated chlorin in which some phenyl groups among the 4 phenyl groups attached to the chlorin ring also remain non-S-glycosylated is obtained.

Preferably, the addition amount of the sugar thiolester is adjusted so that the p-positions of all of the 4 pentafluorophenyl groups linked to a chlorin ring should be S-glycosylated, and the S-glycosylation process is performed in simultaneously using recycling gel filtration chromatography. R group constituting the sugar-SR used includes acyl group and alkyl group.

For the sugar of the thiolester (sugar-SR), hydroxyl groups of the sugar molecule are preferably protected by protecting groups such as acyl. Examples of the protecting group include aliphatic acyl group such as acetyl, pivaloyl; aromatic acyl group such as benzoyl; aralkyl group such as benzyl, and others. Among these protecting groups, acetyl is especially preferred.

When a sugar having hydroxyl groups blocked with protecting groups is used, the protecting group is removed by deprotection performed by a treatment with an alkali or other treatments. In the case where the protecting group is an acyl group, an alkali solution can be added for hydrolysis. For example, a protecting group is removed by treating a protected compound with an alkali like an alkali metal alkoxide such as sodium methoxide, sodium ethoxide, sodium t-butoxide, potassium methoxide, potassium ethoxide, potassium t-butoxide in dichloromethane, methanol, ethanol, tetrahydrofuran or a mixture thereof. When a protecting group is an aralkyl group, it can be removed by hydrogenation with a palladium catalyst. The reason for the deprotection is that sugar residues attached with protecting groups inhibit the drug transferring into cells, therefore, cytotoxic effect can not be obtained.

To prepare a compound wherein a linking group Z or -O-Z- intervenes between a thio group and sugar, sugar thiolester containing a linking group (sugar-Z-SR or sugar-O-Z-SR) may be reacted with a tetrakis(pentafluorophenyl)chlorin derivative.

In the method of production described in the above, porphyrin is converted to chlorin before glycosylation, thus the condition of the conversion process from porphyrin to chlorin can be easily decided. Specifically, sugar was degraded at about 120°C, and above this temperature, the bond between sugar and a porphyrin structure could be cleaved, therefore a reaction which required higher temperature than 120°C was not applicable for the conversion process. From this aspect, before the glycosylation, the conversion process to chlorin can be performed also by a cycloaddition reaction for the pyrrol A ring of a porphyrin skeleton, which generally requires heating above 120°C.
When a derivative of chlorin skeleton is separated and purified from a mixture of a porphyrin skeleton derivative and a chlorin skeleton derivative, the separation and purification of a chlorin derivative without conjugated sugar from a porphyrin derivative without conjugated sugar is easier and its separation and purification efficiency is high, which also contributes to higher productivity, compared with the separation and purification of a glycoconjugated chlorin derivative from a mixture with a glycoconjugated porphyrin derivative.

### [Metal complex-type S-glycosylated chlorin derivatives and their production methods]

The present invention also provides S-glycosylated chlorin derivatives of the following general formula (2) as novel photosensitizers.

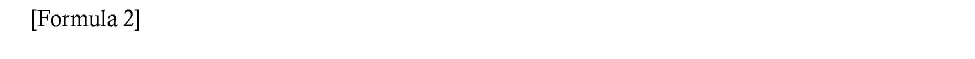

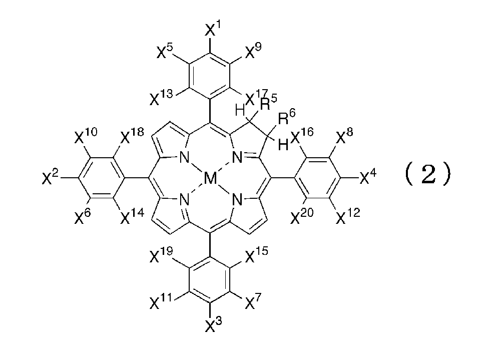

In the formula (2), X¹ to X²⁰, R⁵ and R⁶ are as previously defined for the formula (1).
M represents a metal, specifically, a transition metal, group II, group III and group IV metal, preferably a transition metal such as Pt, Pd, Au, Al and Zn, more preferably a noble metal such as Pt, Pd and Au. Introduction of a metal leads increase in cytotoxicity upon irradiation of light, especially a noble metal introduction makes cytotoxicity significantly increased. Since introduction reaction of a noble metal such as Pt, Pd and Au had to be performed at high temperature, the introduction was difficult because of the presence of sugar in a conventional way. However, the present method of production described in the below has realized the production with high yield.

For the metal complex-type S-glycosylated chlorin derivative of the present invention, preferably, as shown in the formula (2)' below, at least one of X¹, X², X³, and X⁴ in the p-position for the chlorin ring is a group selected from the group consisting of sugar-S-, sugar-Z-S- and sugar-O-Z-S-, and fluorine atoms are attached to the o- and m-positions, and more preferably, X², X², X³, and X⁴ in the p-position are all glycosylated.

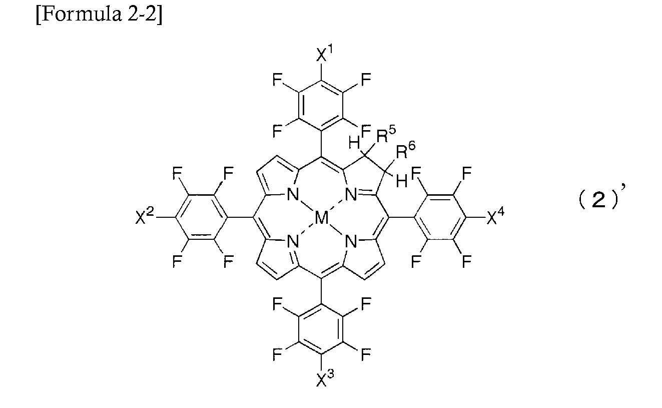

The metal complex of S-glycosylated chlorin derivative constituted as described in the above may be produced by first preparing a S-glycosylated chlorin derivative without metal, followed by a reaction with a metal salt such as hydrochloride or hydrosulfate of an appropriate metal to afford a desired metal complex, however, the following production method of the present invention is preferable from the viewpoint of productivity including stability of sugar, and easiness of purification.

The methods to produce the metal complex of S-glycosylated chlorin derivative of the invention comprise a process to obtain tetraphenylchlorin by subjecting the A ring of tetraphenylporphyrin to a reduction reaction or an addition reaction; a process to obtain a metal complex of tetrakis(pentafluorophenyl)chlorin via a process of reaction of tetraphenylporphyrin or tetraphenylchlorin with a metal salt;
and a process of S-glycosylation by reacting said metal complex of tetrakis(pentafluorophenyl)chlorin with thiolester of sugar or sugar derivatives.

An aromatic ring attached to the chlorin ring can be fluorinated after or before the reaction with a metal salt. In either case, since a metal is introduced before S-glycosylation, the efficiency of isolation of desired metal complex of tetraphenylchlorin from the reaction mixture is high and the productivity of a metal complex of S-glycosylated chlorin derivative is elevated. Furthermore, since the reaction with thiolester of sugar or sugar derivatives is performed after the intended metal complex of tetraphenylchlorin is obtained, the reaction condition for metal introduction and conversion from porphyrin ring to chlorin can be selected from wide range.

More preferably, the porphyrin of tetraphenylporphyrin is first reacted with a metal salt to give a metal complex of tetraphenylporphyrin followed by a reduction reaction or an addition reaction for the A ring of the porphyrin ring to afford tetraphenylchlorin. This mode of production can further improve the yield of a metal complex of tetraphenylchlorin.

In the producing method of a metal complex of S-glycosylated chlorin derivative of the present invention, a porphyrin which is bound with previously fluorinated aromatic rings, i.e. tetrakis(pentafluorophenyl)porphyrin is more preferably used. Thus, the preferred method of producing the metal complex of S-glycosylated chlorin derivative comprises a process of reacting the tetrakis(pentafluorophenyl)porphyrin with a metal salt to give a metal complex of tetrakis(pentafluorophenyl)porphyrin; a process of subjecting the porphyrin A ring of said tetrakis(pentafluorophenyl)porphyrin metal complex to a reduction reaction or an addition reaction to give a tetrakis(pentafluorophenyl)chlorin metal complex; a process of separating and removing the tetrakis(pentafluorophenyl)porphyrin metal complex from the resulting reaction mixture to afford a tetrakis(pentafluorophenyl)chlorin metal complex; and a process of S-glycosylation by reacting said tetrakis(pentafluorophenyl)chlorin metal complex with thiolester of sugar or sugar derivatives.

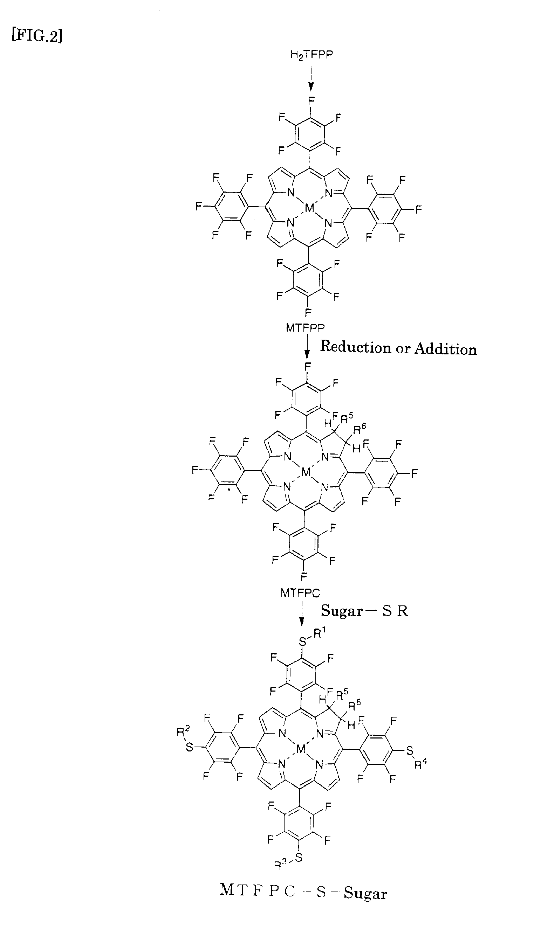

The methods of production of the invention by using tetrakis(pentafluorophenyl)porphyrins are detailed with reference to Fig. 2 in the below.

Firstly, a metal M is introduced into the starting material of 5, 10, 15, 20-tetrakis(pentafluorophenyl)porphyrin (abbr. H₂TFPP). Introduction of the metal M is carried out by reacting its salt with organic acid or inorganic acid such as chloride, bromide, and iodide salts and carboxylate with H₂TFPP.
For example, H₂TFPP and a metal salt to be introduced may be suspended in a suitable organic solvent and heated to reflux, and the resultant reaction mixture may be cooled and concentrated under reduced pressure, then preparative isolation of the desired component may be performed by column chromatography.

The condition of heating can be established according to the kind of metal. In the case of a metal with small ionic radius, such as zinc, approximate heating can be at 30 to 100°C for 5 to 25 hours, however, when a metal with big ionic radius such as Pd and Pt is to be introduced, heating at 200 to 250°C for 80 to 100 hours is required. Since the reaction is performed before the linkage of sugars, the condition may be the one in which only porphyrin skeleton remains stable, thus, such heating at high temperature for long hours can be carried out.

Then, the resultant metal complex of H₂TFPP (abbr. MTFPP) is reduced. The method of reduction can be performed according to the same method of the reduction reaction and the addition reaction which were included in a conversion process from the tetrakis(pentafluorophenyl)porphyrin derivative to the chlorin derivative of the above. In Fig. 2, the resultant tetrakis(pentafluorophenyl)chlorin derivative is represented by MTFPC.

Next, the derivative is S-glycosylated ( In Fig. 2, the resultant S-glycosylated chlorin derivative is represented by MTFPC-S-sugar. This MTFPC-S-sugar has all of the 4 pentafluorophenyl groups being S-glycosylated. R¹, R², R³ and R⁴ are sugar residues derived from sugar-SR). The glycosylation can be performed in the same way as the one in the above production for the S-glycosylated chlorin derivatives. When a protecting group needs to be removed, the method of removing a protecting group is the same as the one used in the above production for the S-glycosylated chlorin derivatives.

To obtain a compound wherein a linking group Z or -O-Z- intervenes between a thio group and a sugar, the sugar thiolester to be reacted for the resulting tetrakis(pentafluorophenyl)chlorin derivative may be an thiolester containing a linking group (sugar-Z-SR or sugar-O-Z-SR).

According to the production method of the invention, metal insertion into the chlorin skeleton is carried out before glycosylation, thus the condition of the metal insertion reaction can be set in a wider range. Conventionally, for the metal complex of O-glycosylated chlorin derivative, only the complex with the metal of which introduction was possible under the heating condition of no more than 100°C could be prepared because of the stability of sugar, however, the production method of the present invention has realized the insertion of a noble metal such as Pt, Pd or the like, which requires heating under reflux at a higher temperature for a longer time, and provided a metal complex with high phototoxicity.

The absorption band of the above described metal complex of S-glycosylated chlorin derivative is shifted to near 600 nm by introduction of a metal, whereas introduction of sugar residues can make the derivative be water soluble and show strong cytotoxicity with irradiation of light but no cytotoxicity in the dark, as is the case with the S-glycosylated chlorin derivative. And its cytotoxicity under irradiation with light is bigger than the one of the O-glycosylated chlorin derivative metal complex, like the case with the S-glycosylated chlorin derivative.

### [Applicability]

The profile of the above mentioned S-glycosylated chlorin derivatives of the present invention, which show strong cytotoxicity under irradiation with light and show no cytotoxicity in the dark, can be used for destroying the target by contacting the derivative with a target biological material in vitro or in vivo in the dark for introducing the derivative into cells, followed by irradiation with light of the absorption wavelength of the chlorin derivative.
The target includes viruses or bacteria, or infected cells thereby, tumor, tumorous tissues, etc. The derivatives have affinity especially for tumor tissue and they can accumulate in tumor area, therefore they can be used for tumor destruction.

Accordingly, S-glycosylated chlorin derivatives and their metal complexes of the present invention can be used as drugs for cancers classified as malignant tumors. Malignant tumors directed in the present invention include not only carcinoma but also sarcoma which is developed in nonepithelial tissue. In particular, the derivatives are useful for the treatment of solid carcinomas in which tumor cells show solid growth in clusters and superficial cancers which light can reach. Specifically, esophagus cancer, lung cancer, stomach cancer, uterin cancer such as cervical cancer and endometrial cancer, skin cancer, prostate cancer and kidney cancer are included. Skin cancer includes primary cancer (squamous cell cancer, basal cell cancer, skin accessory cancer) and also metastasis to skin from internal organ.

The S-glycosylated chlorin derivatives and metal complexes thereof according to the present invention also have affinity not only for malignant tumors, but also for benign tumors, therefore, they can be used as photodynamic drugs for skin diseases such as solar keratoses, skin psoriasis, and so on by local administration.

The characteristic of accumulation to tumors of the S-glycosylated chlorin derivatives of the present invention can be utilized for the diagnosis of cancer in combination with PET, etc.

The maximum absorption wavelength of the S-glycosylated chlorin derivatives of the present invention is 650 nm and it is longer than the one of porphyrin compounds. In particular, the derivatives are water soluble and are expected for penetration into tissue, as a result, they can be applied not only for superficial cancers but also for tumors or solid cancers which are deeply located from the surface. In contrast, the metal complexes of S-glycosylated chlorin derivatives of the present invention have an absorption wavelength near 600 nm, and thus they are suitable for photodynamic drugs and diagnostic reagents for superficial cancers which light can reach and skin disease.

### [Pharmaceutical compositions]

The pharmaceutical composition of the invention comprises the above described S-glycosylated chlorin derivative of the invention (including metal complex type)(as used in this section, both types are collectively referred to as "S-glycosylated chlorin derivatives", unless otherwise specified.) as an active ingredient, and pharmaceutically acceptable carriers of solid or liquid.
The active ingredients, S-glycosylated chlorin derivatives and their metal complexes are both water soluble and show high cell permeability and high phototoxicity, therefore, they can be used appropriately as photodynamic drugs for tumors and solid cancers.

The compound as an active ingredient of the composition of the invention can be administered by a catheter, or intravenous or intramuscular injection, or by other parenteral routes. Subcutaneous administration is also possible. Alternatively, direct local infusion to tumor tissue in the deep part of a body may be performed.

The carriers used in the present invention are suitably selected according to the kind of formulations. An injectable preparation can be formulated into sterile aqueous solution or dispersion, or in a sterile lyophilized form comprising the compound as an active ingredient. For liquid carriers, for example, water, saline, ethanol, hydrous ethanol, glycerol, propylene glycol, and vegetable oil are preferred.

The pharmaceutical composition of the present invention may comprise, together with an active ingredient, diluents such as lactose, sucrose, dibasic calcium phosphate, and carboxymethyl cellulose; lubricants such as magnesium stearate, calcium stearate, and talc; binders such as starch, glucose, syrup, polyvinyl pyrrolidone, cellulose and their derivatives.

Although the dose of the S-glycosylated chlorin derivatives or those metal complexes of the present invention will depend upon the target to be treated or the purpose, the amount of the S-glycosylated chlorin derivative is generally 1 to 3 mg/kg (Photofrin) for tumor diagnosis, 30 to 0.3 mg/kg HpD (Photofrin) for the treatment of tumors, preferably, 1 to 5 mg/kg HpD (Photofrin) or 20 to 0.2 mg/kg (Photofrin).
The S-glycosylated chlorin derivatives or those metal complexes as active ingredients show high cytotoxicity, therefore, it can be expected to obtain equal or stronger effect by a lesser dose than the dose conventionally used. This means that the time required for metabolism and excretion can be shortened, and convenience in photodynamic therapy can be improved.

For tumor treatment, the S-glycosylated chlorin derivative or its metal complex is administered, and then the light covering the absorption band of said compound is irradiated to the site of treatment. Specifically, photoradiation at a wavelength of more than 500 nm can produce singlet oxygen to develop the intended cytotoxicity, preferably photoradiation with light having the maximum absorption wavelength in higher proportion is carried out.
Light source includes laser and halogen lamp. Any lasers including dye laser, semiconductor laser and argon laser can be used for producing light of the wavelength required for excitation.

S-glycosylated chlorin derivatives of the present invention have big absorption coefficient, therefore the strength of light to be irradiated can be weaker than the strength used for a conventional photosensitizer. Thus, photodynamic therapy using the tetraphenylchlorin derivative or its metal complex of the present invention can reduce a burden on a body receiving the therapy. Specifically, the amount of the photoradiation is 1 to 20 J/cm².

### EXAMPLES

### [Production of S-glucosylated chlorin derivative]

### Example 1: S-glucosylated chlorin derivative

### (1) Preparation of H₂TFPP (Synthesis of tetrakis(pentafluorophenyl)porphyrin derivative)

Pentafluorobenzaldehyde (5 g) and pyrrole (2 mL) were dissolved in dichloromethane (1.2 L) under substitution with nitrogen. To the resultant solution was added boron trifluoride ether complex (1 mL) and heated under reflux for 4 hours, followed by addition of chloranil (6.9 g), and heated under reflux for another 12 hours. The resulting reaction mixture was cooled, concentrated under reduced pressure, and then loaded onto a silica gel column, and eluted with chloroform for preparative isolation to give 5, 10, 15, 20-tetrakis(pentafluorophenyl)porphyrin (abbr. H₂TFPP). After concentration under reduced pressure, it was recrystallized from the mixture of chloroform and methanol (yield: 3.8 g; 61%).

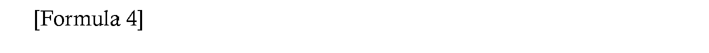

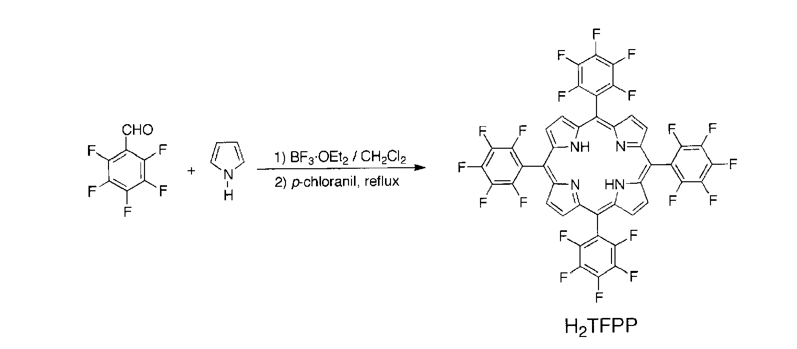

### (2) Preparation of H₂TFPC (Conversion from porphyrin derivative to chlorin derivative)

H₂TFPP (892 mg) prepared in (1), N-methylglycine (171 mg) and paraformaldehyde (134 mg) were dissolved in toluene (200 mL). The resulting solution was heated at reflux under nitrogen atmosphere for 20 hours while N-methylglycine (ca. 400 mg) and paraformaldehyde (400 mg) were added at every 2 hours. After the reaction mixture was cooled, the solvent was distilled off under reduced pressure. Chloroform (50 mL) was added, and washed with water to remove excessive amounts of N-methylglycine and paraformaldehyde. The toluene solution was dried with anhydrous sodium sulfate, and then the solvent was distilled off under reduced pressure. The resulting crude product was loaded onto a silica gel column, and eluted with chloroform for preparative isolation to give 5, 10, 15, 20-tetrakis(pentafluorophenyl)-2,3-[methano(N-methyl)iminomethano]chlorin (abbr. H₂TFPC), which is further concentrated under reduced pressure (yield: 595 mg; 55%).

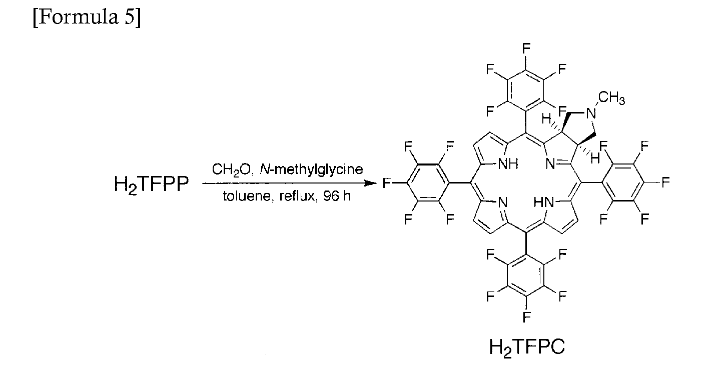

### (3) Preparation of H₂TFPC-SAcGlc (S-glucosylation)

H₂TFPC (102 mg) obtained in (2), 2, 3, 4, 6-tetra-O-acetyl-β-D-glucopyranosylthioacetyl (abbr. AcGlcSAc) (375 mg) and diethylamine (2 mL) were dissolved in dimethylformamide (20 mL), and stirred at room temperature for 16 hours. To the reaction mixture was added chloroform (20 mL), and the mixture was washed with water. The organic layer was dried with anhydrous sodium sulfate, and then the solvent was distilled off under reduced pressure. The resultant crude product was loaded onto a silica gel column, and eluted with a mixture of chloroform and methanol, and gel permeation preparative chromatography was also performed to give 5, 10, 15, 20-tetrakis[4-(2, 3, 4, 6-tetra-O-acetyl-β-D-glucopyranosylthio)-2, 3, 5, 6-tetrafluorophenyl]-2, 3-[methano(N-methyl)iminomethano]chlorin (abbr. H₂TFPC-SAcGlc) (yield: 210 mg; 94%).

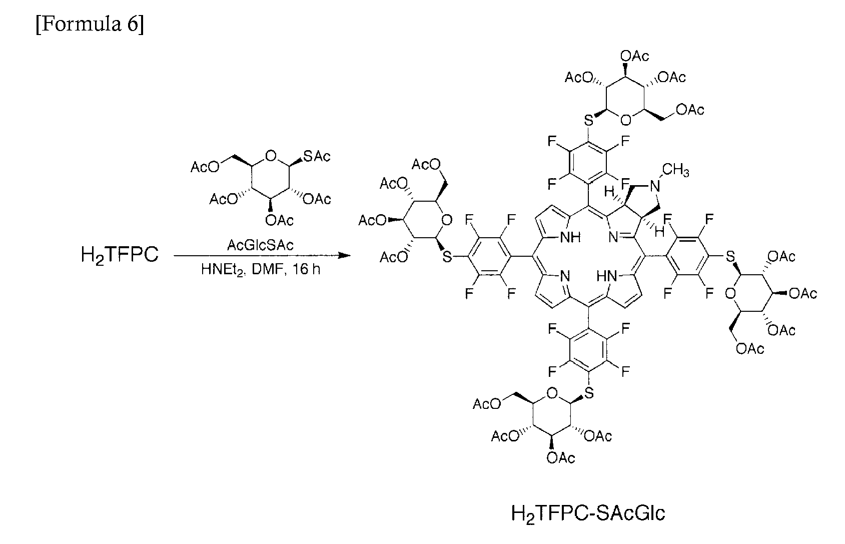

### (4) Preparation of H₂TFPC-SGlc (Removal of acetyl protecting group)

H₂TFPC-SAcGlc (185 mg) was dissolved in a mixture of dichloromethane (20 mL) and methanol. To the resulting solution was added sodium methoxide till the pH reached 9, and then the solution was heated at reflux under nitrogen atmosphere at 15°C to 20°C for 12 hours and in hot-water bath at 45°C to 50°C for 3 hours. The resulting reaction mixture was cooled and then neutralized with acetic acid, and concentrated under reduced pressure. The resulting precipitation was filtered, and the residue was washed, and then dried in vacuo to give 5, 10, 15, 20-tetrakis[4-(β-D-glucopyranosylthio)-2, 3, 5, 6-tetrafluorophenyl]-2, 3-[methano(N-methyl)iminomethano]chlorin (abbr. H₂TFPC-SGlc) (yield: 81 mg; 47%). ¹⁹F-NMR spectrum confirmed that the resulting compound was S-glycosylated in the p-position of all the phenyl groups.

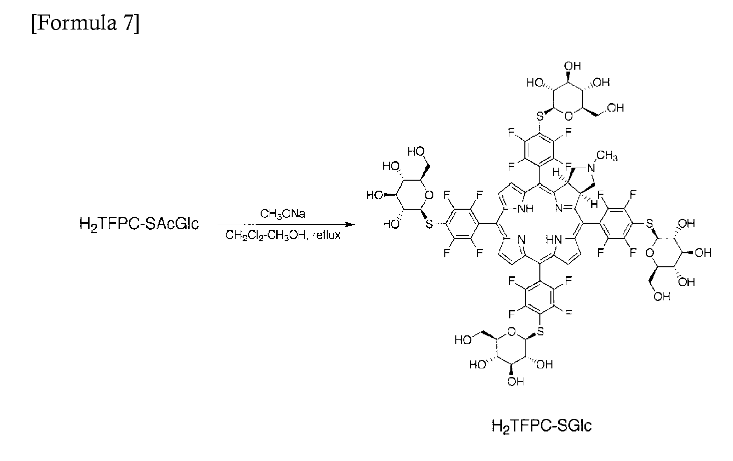

### Example 2: Pd complex of H₂TFPC-SGlc

### (1) Preparation of PdTFPP (Introduction of metal)

H₂TFPP (1 g) prepared in Example 1, (1) and palladium chloride (0.4 g) were dissolved in benzonitrile (35 mL). After the atmosphere was substituted with nitrogen, the resulting solution was heated at reflux under nitrogen at 220°C for 96 hours. The resultant reaction mixture was cooled and then concentrated under reduced pressure. The resulting crude product was loaded onto an alumina column and eluted with chloroform for preparative isolation. Further isolation was performed by silica gel chromatography and the resulting product was concentrated under reduced pressure, followed by recrystallization from a mixture of dichloromethane and methanol to provide [5, 10, 15, 20-tetrakis(pentafluorophenyl)porphyrinato]palladium (II) (abbr. PdTFPP) (yield: 0.76 g; 70%).

### (2) Preparation of PdTFPC (Conversion from porphyrin ring to chlorin ring)

PdTFPP (0.4 g) prepared in (1), N-methylglycine (0.2 g) and paraformaldehyde (0.17 g) were dissolved in toluene (50 mL) and the PdTFPP was converted to chlorin skeleton by a cycloaddition reaction for the A ring of the porphyrin ring according to the conversion process of Example 1 (2). And then it was subjected to silica gel chromatography and concentrated under reduced pressure, and the target component was further separated by gel permeation chromatography followed by recrystallization from a mixture of chloroform and hexane to provide {5, 10, 15, 20-tetrakis(pentafluorophenyl)-2, 3-[methano(N-methyl)iminomethano]chlorinato}palladium (abbr. PdTFPC) (yield: 0.075 g; 17%).

### (3) Preparation of PdTFPC-SAcGlc (S-glucosylation)

PdTFPC (0.11 g) obtained in (2), AcGlcSAc (0.19 g) and diethylamine (3 mL) were dissolved in dimethylformamide (30 mL), and S-glycosylation was performed according to Example 1 (3). From the resultant crude product, the target component was separated by gel permeation preparative chromatography, and the solvent was distilled off under reduced pressure to give {5, 10, 15, 20-tetrakis[4-(2, 3, 4, 6-tetra-O-acetyl-β-D-glucopyranosylthio)-2, 3, 5, 6-tetrafluorophenyl]-2, 3-[methano(N-methyl)iminomethano]chlorinato}palladium (II) (abbr. PdTFPC-SAcGlc) (yield: 0.15 g; 66%).

### (4) Preparation of PdTFPC-SGlc (Removal of acetyl protecting group)

PdTFPC-SAcGlc (0.15 g) prepared in (3) was dissolved in a mixture of dichloromethane (20 mL) and methanol (20 mL), the acetyl groups as protecting groups for hydroxyl groups of glucose were removed in accordance with Example 1 (4), and the target component was separated by reversed phase liquid chromatography, and the solvent was distilled off under reduced pressure, and then the residue was desalted by using a filtration membrane with a molecular weight cutoff of 1000, and the solvent distilled off to provide {5, 10, 15, 20-tetrakis[4-(β-D-glucopyranosylthio)-2, 3, 5, 6-tetrafluorophenyl]-2, 3-[methano(N-methyl)iminomethano]chlorinato}palladium (II) (abbr. PdTFPC-SGlc) (yield: 0.065 g; 59%). ¹⁹F-NMR spectrum confirmed that the obtained compound was S-glycosylated at the p-positions in all of the phenyl groups.

### Example 3: H₂TFPC-SGal

Example 1 was followed except that 2, 3, 4, 6-tetra-o-acetyl-β-D-galactopyranosylacetylthioacetyl (abbr. AcGalSAc) was used instead of AcGlcSAc in the S-glucosylation process of Example 1 (3) to give 5, 10, 15, 20-tetrakis[4-(β-D-galactopyranosylthio)-2, 3, 5, 6-tetrafluorophenyl]-2, 3-[methano(N-methyl)iminomethano]chlorin of which sugar is galactose (abbr. H₂TFPC-SGal)
(yield: 86 mg; 84%).

### [Preparation of porphyrin derivative]

### Comparative Example 1: H₂TFPP-SGlc

H₂TFPP (55 mg) prepared in Example 1 (1), AcGlcSAc (207 mg) and diethylamine (0.8 mL) were dissolved in dimethylformamide (10 mL), stirred at room temperature for 24 hours, and the procedure of Example 1 (3) was followed for S-glycosylation and gel permeation preparative chromatography was performed for separation, then the solvent was distilled off under reduced pressure to provide 5, 10, 15, 20-tetrakis(4-(2, 3, 4, 6-tetra-O-acetyl-β-D-glucopyranosylthio)-2, 3, 5, 6-tetrafluorophenyl)porphyrin (abbr. H₂TFPP-SAcGlc) (yield: 100 mg; 76%).

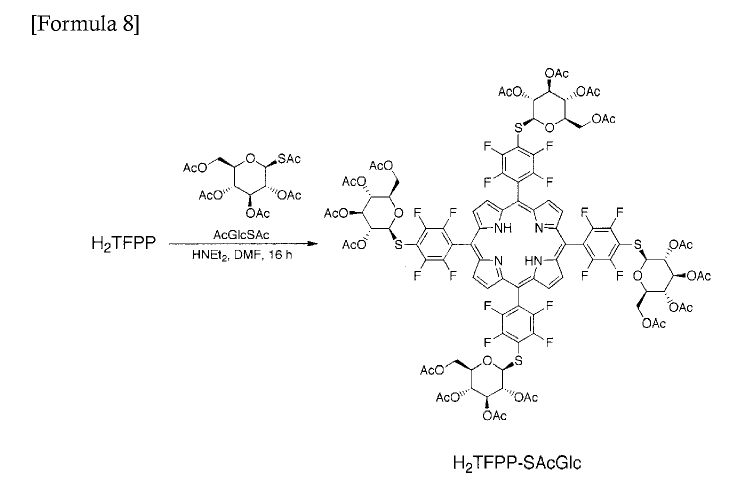

The resulting H₂TFPP-SAcGlc (152 mg) was dissolved in a mixture of dichloromethane (20 mL) and methanol (20 mL), and the procedure of Example 1 (4) was followed to remove acetyl groups, then it was subjected to reversed phase liquid chromatography to fractionate the target component. The solvent was distilled off from the fractioned solution under reduced pressure to give 5, 10, 15, 20-tetrakis[4-(β-D-glucopyranosylthio)-2, 3, 5, 6-tetrafluorophenyl]porphyrin (abbr. H₂TFPP-SGlc) (yield: 56 mg; 37%).

### Comparative Example 2: PdTFPP-SGlc

After PdTFPP (77 mg) prepared in Example 2 (1), AcGlcSAc (224 mg) and diethylamine (1.2 mL) were dissolved in dimethylformamide (10 mL), the procedure of Example 1 (3) was followed for S-glycosylation, and separation was performed by gel permeation preparative chromatography, then the solvent was distilled off under reduced pressure to provide {5, 10, 15, 20-tetrakis[4-(2, 3, 4, 6-tetra-O-acetyl-β-D-glucopyranosylthio)-2, 3, 5, 6-tetrafluorophenyl]porphyrinato}palladium (II) (abbr. PdTFPP-SAcGlc) (yield: 157 mg; 89%).

The resulting PdTFPP-SAcGlc (184 mg) was dissolved in a mixture of dichloromethane (20 mL) and methanol (20 mL), and the procedure of Example 1 (4) was followed to remove acetyl groups, and thereafter reversed phase liquid chromatography was performed to fractionate the target component. The solvent was distilled off from the fractionated solution under reduced pressure to give {5, 10, 15, 20-tetrakis[4-(β-D-glucopyranosylthio)-2, 3, 5, 6-tetrafluorophenyl]porphyrinato}palladium (II) (abbr. PdTFPP-SGlc) (yield: 46 mg; 25%).

### [O-glycosylated chlorin derivative]

### Comparative Example 3: m-TGlcPC

According to the method described in WO 2002-20536, production of 5, 10, 15, 20-tetrakis[3-(2', 3', 4', 6'-tetra-O-acetyl-β-D-glucopyranosyloxy)phenyl]porphyrin (abbreviated as TGlcPP in the below) was carried out, and then the A ring of the porphyrin ring was reduced to give 5, 10, 15, 20-tetrakis[3-(β-D-glucopyranosyloxy)phenyl]chlorin (abbr. m-TGlcPC) represented by the following formula.

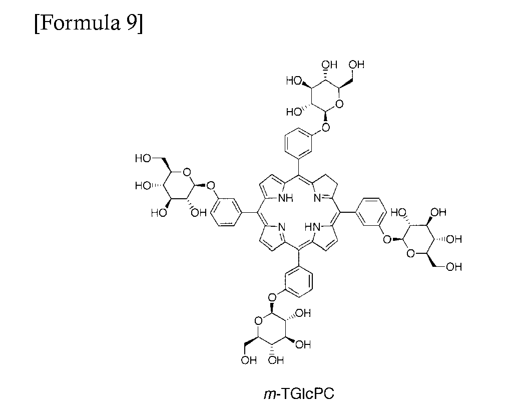

### Reference Example 1: PtTFPC

Sodium chloroplatinate was used instead of palladium chloride in Example 2 (1) to provide PtTFPP, and the same procedure of the Example was carried out to convert the porphyrin ring to a chlorin ring to obtain PtTFPC.

### Reference Example 2:

Commercially available 5, 10, 15, 20-tetrakis(4-sulfophenyl)porphyrin (abbr. TPPS) was used.

### [Evaluation of phototoxicity]

HeLa cells were plated in a 96-well plate to give a concentration of 5 × 10³ cells/well (medium volume: 100 µL), and the plate was incubated at 37°C in 5% CO₂ atmosphere for 24 hours. To each well was added 100 µL of a drug solution containing 2% dimethylsulfoxide to give a certain concentration. The plate was further incubated at 37°C in 5% CO₂ atmosphere for 24 hours. Then, the medium was discarded and the cells were washed twice with phosphate buffered saline (100 µL). To each well was added 100 µL of Dulbecco's modified Eagle's medium (DMEM) containing 10% fetal calf serum (FCS). The cells were then exposed to light (16 J/cm²) from a 100 W halogen lamp equipped with a 500 nm cut-off filter and a water jacket. After that, the plate was further incubated at 37°C in 5% CO₂ for 24 hours. And the plate was subjected to WST-8 assay and cell viability rates were calculated as relative values to those obtained in the absence of drugs. The average values of the result from 6 replicate experiments for each drug were calculated.
The light dose was adjusted by controlling the irradiation time by using a power meter.
WST assay is one of the method to determine the activity of dehydrogenase of cells, in which tetrazolium salt (WST-8) is reduced dependently on the number of viable cells. The amount of the reduction was determined by absorption spectroscopy to provide the relative amount of viable cells.

### [Comparison of phototoxicity]

### (1) Drug concentration of 0.5 µM

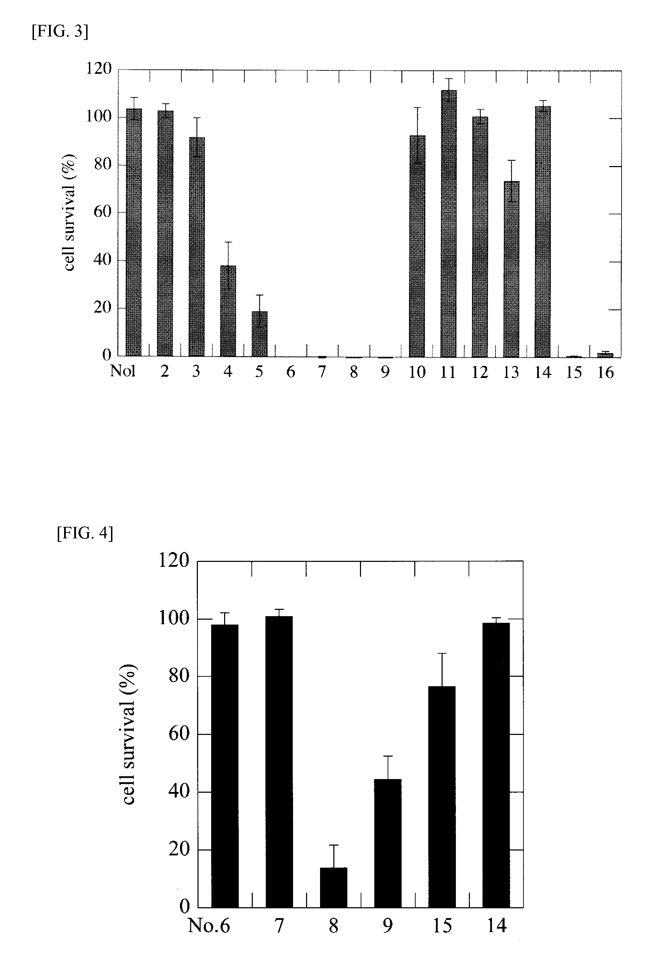

Phototoxicity were determined for the chlorin derivatives or porphyrin derivatives which were prepared in the above Example and Comparative Example and obtained as production intermediates, and the compound from Reference Example at a drug concentration of 0.5 µM according to the above evaluation method. The result is shown in Fig. 3. Each compound corresponding to the compound No. in Fig. 3 is listed in Table 1.

**[Table 1]**

| Compound No. | Compound |
|---|---|
| 1 | H₂TFPP |
| 2 | PdTFPP |
| 3 | H₂TFPC |
| 4 | PdTFPC |
| 5 | PtTFPC |
| 6 | H₂TFPP-SGlc |
| 7 | PdTFPP-SGlc |
| 8 | H₂TFPC-SGlc |
| 9 | PdTFPC-SGlc |
| 10 | H₂TFPP-SAcGlc |
| 11 | PdTFPP-SAcGlc |
| 12 | H₂TFPC-SAcGlc |
| 13 | PdTFPC-SAcGlc |
| 14 | TPPS |
| 15 | m-TGlcPC |
| 16 | H₂TFPP-SGal |

In Fig. 3, when each compound No. 1, 2, 3 and 4 was compared respectively with the compound No. 6, 7, 8 and 9, it was revealed that the glycoconjugated compound shows higher phototoxicity in either case. In the comparison of the compound No. 6, 7, 8 and 9 respectively with the compound No. 10, 11, 12 and 13, it was found that the phototoxicity was disappeared in compounds having hydroxyl groups of sugars protected with acetyl groups. Moreover, in the comparison of the compound No. 3 with No. 4 and 5, it was found that introduction of a metal into a chlorin derivative made the phototoxicity increased.

In contrast, S-glucosylated chlorin derivatives (No. 8 and 9), S-glucosylated porphyrin derivatives (No. 6 and 7), O-glucosylated chlorin derivative (No. 15), S-galactosylated chlorin derivative (No. 16) each showed higher phototoxicity compared with commercially available TPPS (No. 14).

### (2) Drug concentration of 0.09 µM

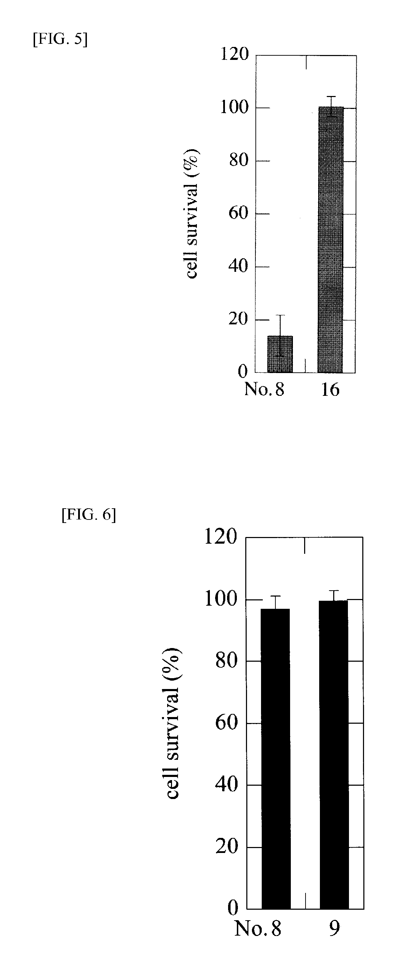

For the compounds No. 6 to 9 and 14 to 16 shown in Table 1, phototoxicity was determined at a drug concentration of 0.09 µM according to the above evaluation method. The results are shown in Fig. 4 and Fig. 5.

At the drug concentration of 0.5 µM, when S-glucosylated chlorin derivatives (No. 8 and 9) were compared with S-glucosylated porphyrin derivatives (No. 6 and 7) and O-glucosylated chlorin derivative (No. 15), no difference in phototoxicity was found, however, at 0.09 µM concentration of drug as shown in Fig. 4, there existed significant difference between glycoconjugated chlorin derivatives and glycoconjugated porphyrin derivatives. Thus, as is obvious from the comparison of the compounds No. 6, 7 and 14 with No. 8 and 9, at the concentration of 0.09 µM, sugar linkage to the porphyrin derivatives did not result in any improvement effect of phototoxicity, however, sugar linkage to the chlorin derivatives led distinguished improvement of phototoxicity.

From the comparison of the compounds No. 8 and 9 with 15 at the concentration of 0.5 µM, there were not any differences in phototoxicity for those glycoconjugated chlorin derivatives, however, at 0.09 µM, there was a difference in phototoxicity between the S-glycosylated chlorin derivatives and the O-glycosylated chlorin derivative, and those S-glycosylated chlorin derivatives (No. 8 and 9) showed higher phototoxicity.

Furthermore, from Fig. 5 as a result of the experiment at the drug concentration of 0.09 µM, it was found that S-glycosylated chlorin derivatives for which glucose was linked as a sugar (No. 8) exhibited higher phototoxicity than the derivative for which galactose was linked (No. 16), the amount of the derivative required to obtain the same level of phototoxicity was smaller in glucose-conjugated chlorin derivatives.

### [Comparison of cytotoxicity in the dark]

For the compound (No. 8 and 9) prepared in the above Example 1 and 2, cell survival was studied without irradiation of light (light intensity: 0 J/cm²) according to the phototoxicity evaluation method. The result is shown in Fig. 6.
Fig. 6 revealed that each compound did not show any cytotoxicity in the dark.

### [Production of chlorin derivative with linking groups]

### Example 4: H₂TFPC-SCH₂CH₂OAcGlc

Example 1 was followed except that 2-(S-acetyl)mercaptoethyl 2, 3, 4, 6-tetra-O-acetyl-β-D-glucopyranoside (AcGlcOCH₂CH₂SAc) was used instead of 2, 3, 4, 6-tetra-O-acetyl-β-D-glucopyranosylthioacetyl (AcGlcSAc) in the S-glucosylation process in Example 1 (3) to provide a S-glycosylated chlorin derivative of the following structure.

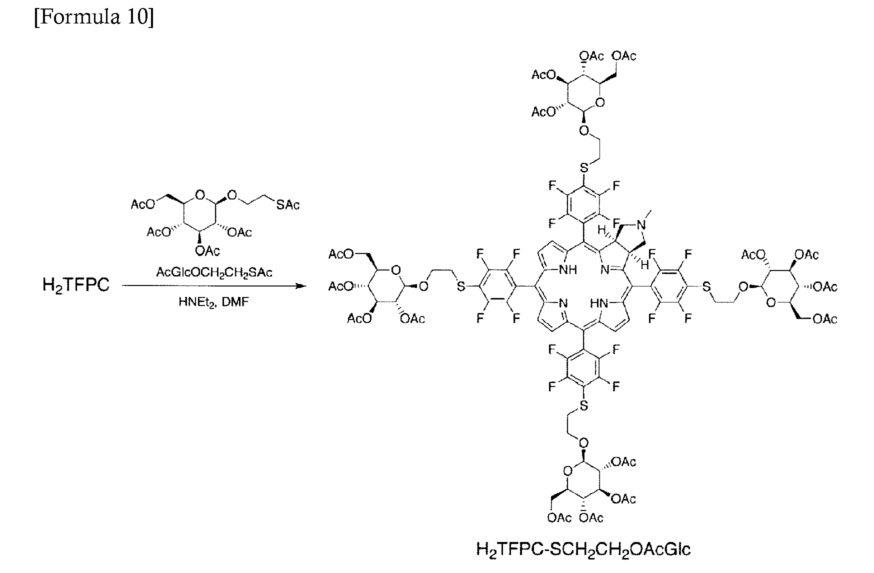

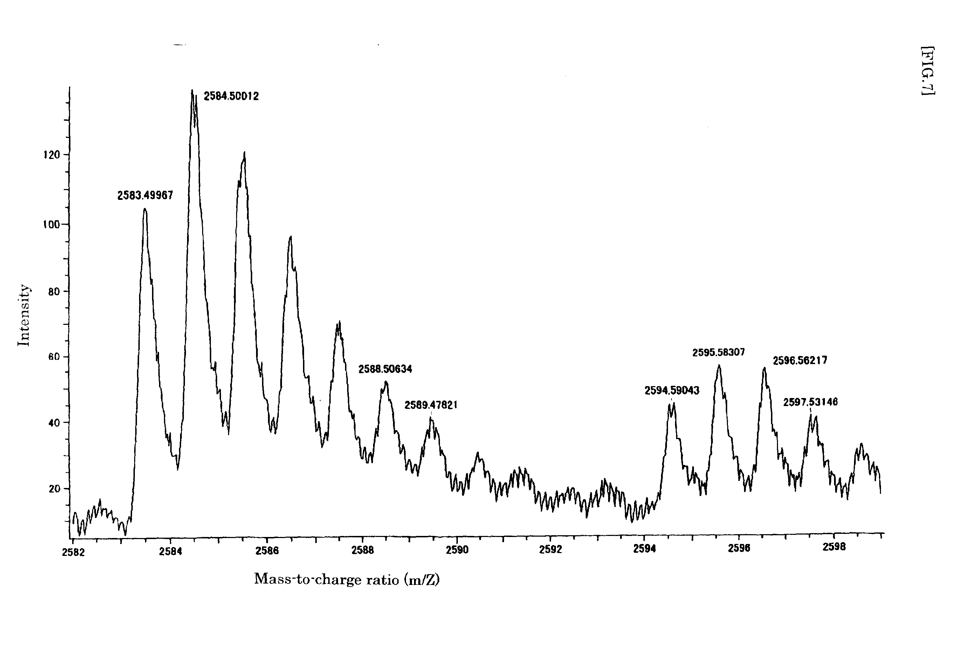

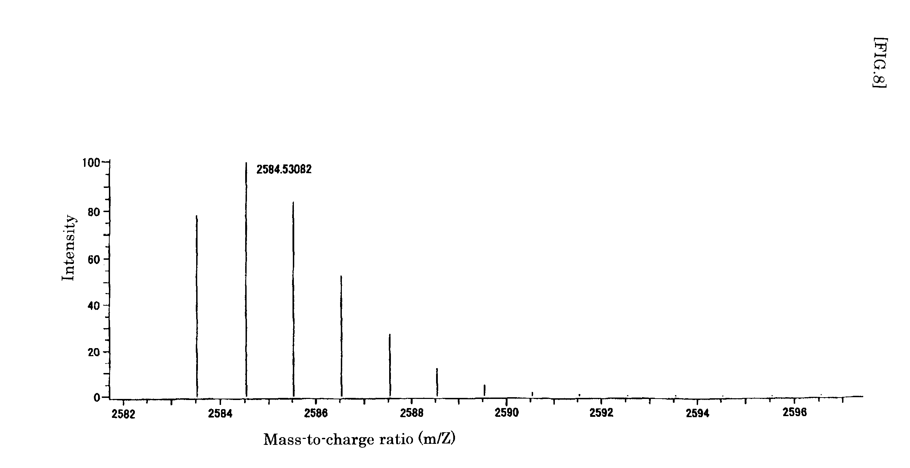

The spectrum of the resulting H₂TFPC-SCH₂CH₂OAcGlc was measured by ESI-MS (ionization mode ESI, spectrum recording interval: 1.0 sec, orifice voltage sweep: 200V). The spectrum obtained is shown in Fig. 7. The peak (2584.50012) corresponding to 2585 average mass of the target compound was confirmed. The mass spectrum shown in Fig. 7 and the simulated mass spectrum of H₂TFPC-SCH₂CH₂OAcGlc (ref. Fig. 8) showed almost the same main peaks (2583.5, 2588.5, 2589.5). Thus, it was confirmed that H₂TFPC-SCH₂CH₂OAcGlc was prepared. By removing acetyl protecting groups in the same manner of Example 1, the target chlorin derivative can be prepared.

### [Production of other chlorin derivatives]

### Example 5: H₂TFPC'-SAcGlc

1g of H₂TFPP prepared in the above was dissolved in 20 ml of toluene, and 9.76 mL of benzyl bromide, 24 mL of tributyltin hydride, and 1.7 g of azoisobutylonitrile were added under heating at 60°C for a period of 17 days. The target component was fractionated from the crude product by silica gel column chromatography with hexane as an eluting solution, and the target component was collected by gel permeation preparative chromatography. Recrystallization was also performed from chloroform and acetonitrile to give 35 mg of 10, 15, 20-tetrakis(pentafluorophenyl)-2-benzyl chlorin (abbr. H₂TFPC') of the following structure. 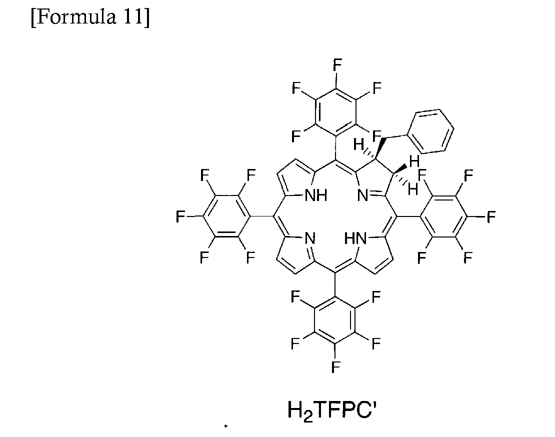 S-glucosylation was performed for H₂TFPC' in the same manner of Example 1 to give S-glycosylated chlorin derivative (HTFPC'-SAcGlc) of the following structure.

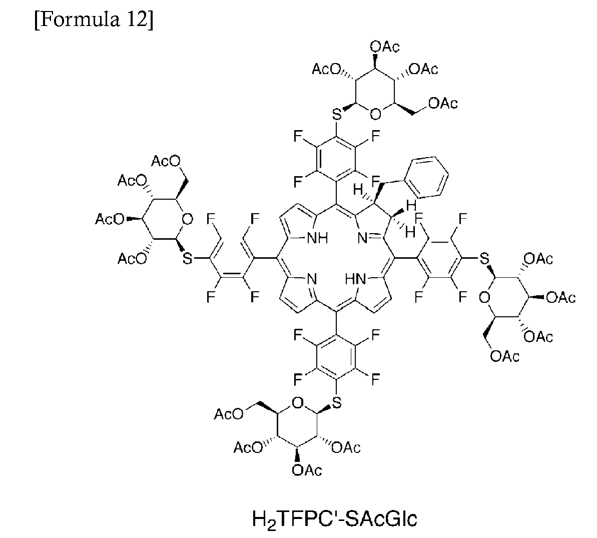

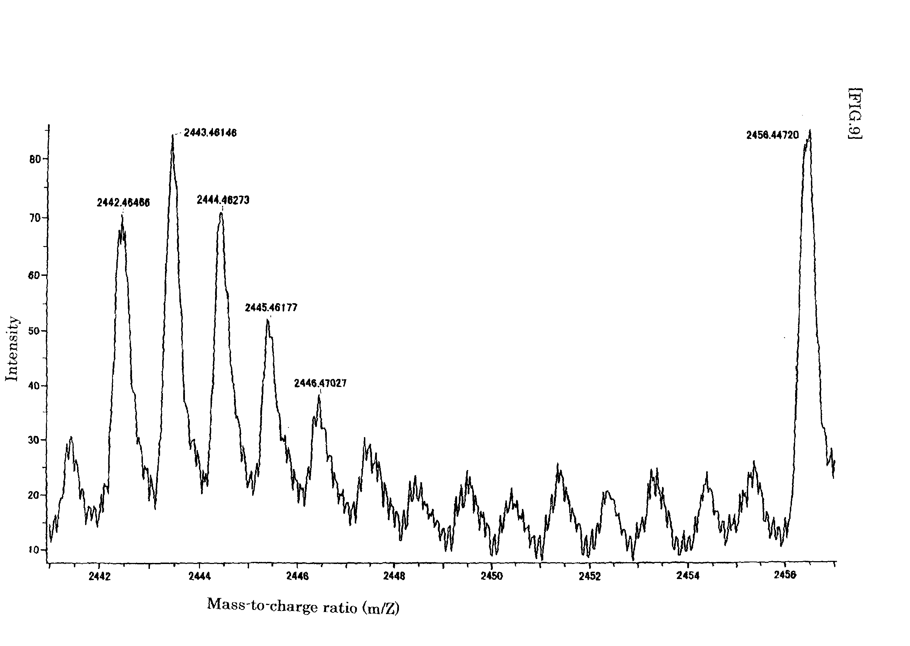

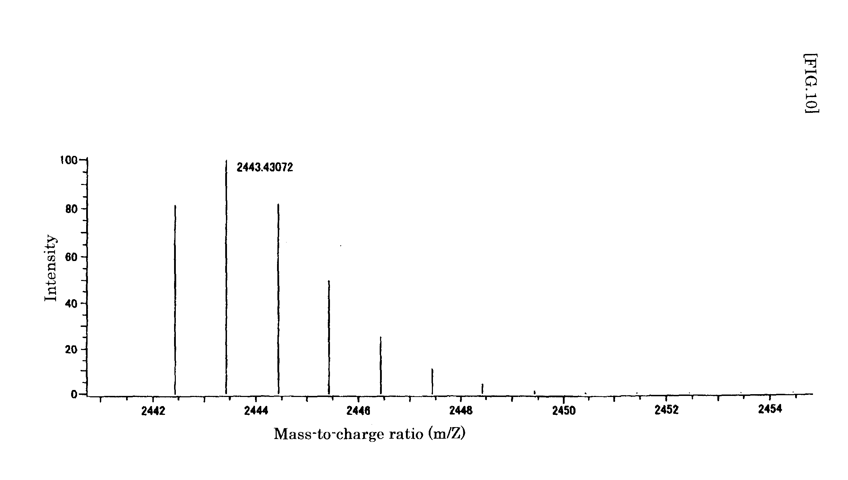

The spectrum of the resulting HTFPC'-SAcGlc was measured by ESI-MS (ionization mode ESI, spectrum recording interval: 1.0 sec, orifice voltage sweep: 150V). The spectrum obtained is shown in Fig. 9. The peak (2443.43072) which corresponds to 2444.13, theoretical average mass of the target compound was confirmed. The mass spectrum shown in Fig. 9 and the simulated mass spectrum of HTFPC'-SAcGlc (ref. Fig. 10) showed almost the same main peaks (2442.5, 2444.5, 2445.5, 2446.5), which confirmed that HTFPC'-SAcGlc was prepared. By removing acetyl protecting groups in the same manner of Example 1, the target chlorin derivative can be prepared.

### Example 6: S-glucosylated chlorin derivative linked with 5 molecules of sugar (H₂TFPC-SGlc₅)

The byproduct obtained in the production of H₂TFPC-SAcGlc in Example 1 was isolated and purified by silica gel chromatography (developing solvent (ethyl acetate: hexane = 3: 2), H₂TFPC-SAcGlc (Rf value = 0.18), H₂TFPC-SAcGlC₅ (Rf value = 0.11)). The target fraction was determined by ESI-MS to confirm that there was no peak resulted from H₂TFPC-SAcGlc, and only the peaks of 2751.53 and 2773.8747 corresponding to 2752.51 and 2774.49 of the average masses of the target compound (H₂TFPC-SAcGlc₅) were found and it was revealed that the S-glucosylated chlorin derivative, in which 5 molecules of glucose were linked within one molecule, was obtained.

The resulting H₂TFPC-SAcGlc₅ (479 mg) was dissolved in a mixture of dichloromethane (30 ml) and methanol (30 ml) and then sodium methoxide (188 mg) was added and stirring continued at 25°C under nitrogen for 24 hours. The resulting reaction mixture was cooled and then nutralized with acetic acid and concentrated under reduced pressure. The precipitate obtained was filtered, and the residue was washed, and then dried in vacuo to give H₂TFPC-SGlc₅ (yield: 326 mg). The determination by ESI-MS revealed the peaks (1911.58 and 1933.55) corresponding to the average masses of H₂TFPC-SGlc₅ (1912.30 and 1934.28), and the compound was identified as H₂TFPC-SGlc₅.

### [Production of other metal complexes of S-glycosylated chlorin]

### Example 7:

To a methanol solution of H₂TFPC-SGlc prepared in Example 1 was added zinc chloride and the mixture was reacted at 30°C for 24 hours. After the reaction, the spectrum was determined by ESI-MS, in which the peak of 1758 m/z mainly found in Na solution of H₂TFPC-SGlc became lower and a peak of 1820.4 was appeared. This peak coincided mostly with the peak of the theoretical average mass of H₂TFPC-SGlc zinc complex.

### Example 8:

H₂TFPC-SGlc prepared in Example 1 and manganese chloride were reacted in a mixture of methanol (10 ml) and water (10 ml) at 80°C for 24 hours. After the reaction, the spectrum was determined by ESI-MS and a peak of 1788.29 was appeared. This peak almost coincided with the theoretical average mass of H₂TFPC-SGlc manganese complex.

### INDUSTRIAL APPLICABILITY

The novel glycoconjugated chlorin derivatives and their metal complexes of the present invention are water soluble and have high tissue permeability and show strong phototoxicity by irradiation with light, therefore, they are useful as effective components for PDT drugs. Moreover, novel glycoconjugated chlorin derivatives of the present invention have strong absorption peaks in the longer wavelength area than conventional drugs, thus a range of application can be expanded from those of conventional PDT drugs.

## Claims

1. A S-glycosylated chlorin derivative represented by the following general formula (1), wherein X¹ to X²⁰ independently one another represent a group selected from the group consisting of F-, sugar-S-, sugar-Z-S- and sugar-O-Z-S-, and at least one among X¹ to X²⁰ represents a group selected from the group consisting of sugar-S-, sugar-Z-S-, and sugar-O-Z-S-(where F represents a fluorine atom, S represents a sulfur atom, O represents an oxygen atom, and Z represents a hydrocarbon group having 1 to 6 carbon atoms); and R⁵ and R⁶ independently represent a hydrogen atom or an organic group or may together form a ring.

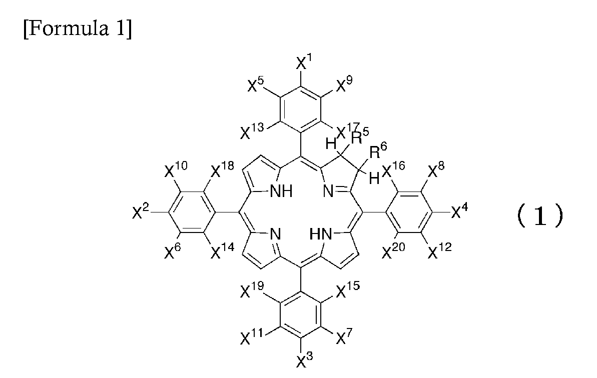

2. A S-glycosylated chlorin derivative represented by the following general formula (1)', wherein X¹, X², X³, and X⁴ independently one another represent a group selected from the group consisting of F-, sugar-S-, sugar-Z-S- and sugar-O-Z-S-, and at least one among X¹ to X²⁰ represents a group selected from the group consisting of sugar-S-, sugar-Z-S-, and sugar-O-Z-S-(where F represents a fluorine atom, S represents a sulfur atom, O represents an oxygen atom, and Z represents a hydrocarbon group having 1 to 6 carbon atoms); and R⁵ and R⁶ independently represent a hydrogen atom or an organic group or may together form a ring.

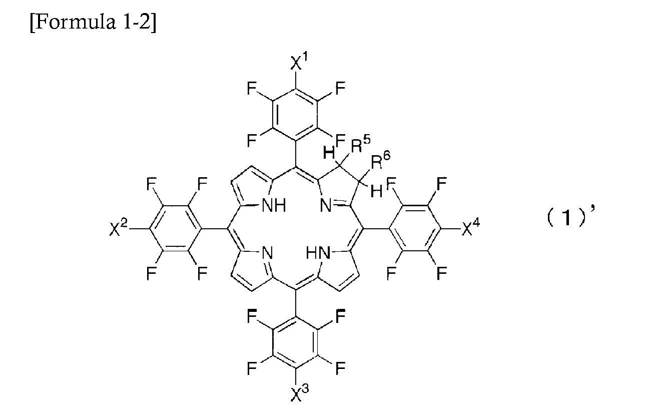

3. A chlorin derivative according to claim 2, wherein all of X¹, X², X³, and X⁴ in the formula (1)' are sugar-S-.

4. A S-glycosylated chlorin derivative represented by the following general formula (2), wherein X¹ to X²⁰ independently one another represent a group selected from the group consisting of F-, sugar-S-, sugar-Z-S- and sugar-O-Z-S-, and at least one among X¹ to X²⁰ represents a group selected from the group consisting of sugar-S-, sugar-Z-S-, and sugar-O-Z-S-(where F represents a fluorine atom, S represents a sulfur atom, O represents an oxygen atom, and Z represents a hydrocarbon group having 1 to 6 carbon atoms); R⁵ and R⁶ independently represent a hydrogen atom or an organic group or may together form a ring; and M represents a metal.

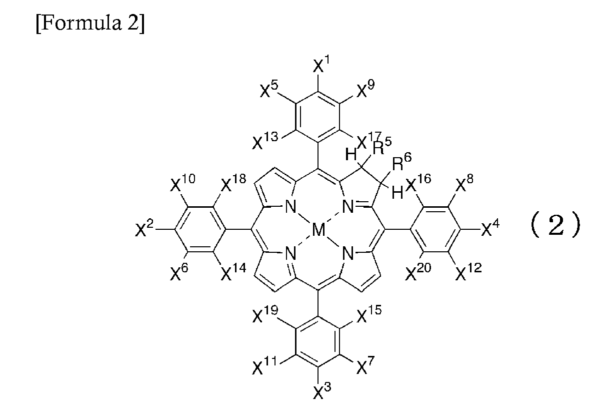

5. A S-glycosylated chlorin derivative represented by the following general formula (2)', wherein X¹, X², X³, and X⁴ independently one another represent a group selected from the group consisting of F-, sugar-S-, sugar-Z-S- and sugar-O-Z-S-, and at least one among X¹, X², X³, and X⁴ represents a group selected from the group consisting of sugar-S-, sugar-Z-S-, and sugar-O-Z-S- (where F represents a fluorine atom, S represents a sulfur atom, O represents an oxygen atom, and Z represents a hydrocarbon group having 1 to 6 carbon atoms); R⁵ and R⁶ independently represent a hydrogen atom or an organic group or may together form a ring; and M represents a metal.

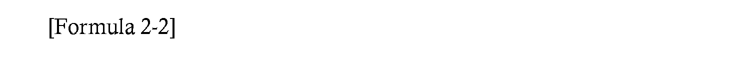

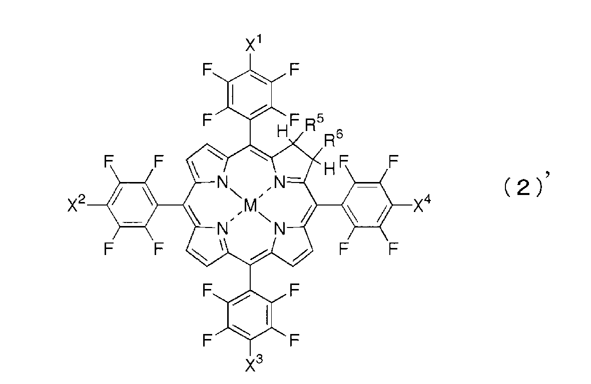

6. A chlorin derivative according to claim 5, wherein all of X¹, X², X³, and X⁴ in the formula (2)' are sugar-S-.

7. A chlorin derivative according to any one of claims 4 to 6, wherein the M is a transition metal.

8. A chlorin derivative according to any one of claims 4 to 6, wherein the M is one selected from the group consisting of Pt, Pd, and Au.

9. A S-glycosylated chlorin derivative according to any one of claims 1 to 8, wherein the organic group is lower alkyl or Q-(CH₂)ₖ- (where Q represents lower alkyl, aryl, amino, lower alkylamino, imino, lower alkylimino, or cycloalkyl group, and k is an integer of 0 to 3).

10. A S-glycosylated chlorin derivative according to any one of claims 1 to 9, wherein the sugar is selected from the group consisting of monosaccharide, disaccharide, and polysaccharide.

11. A chlorin derivative according to claim 10, wherein the sugar is pentose or hexose represented by the following formulae.

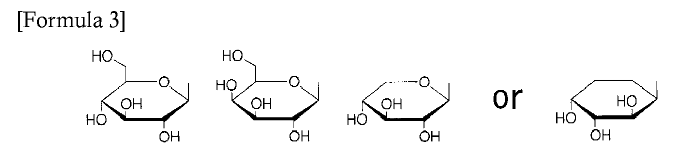

12. A chlorin derivative according to claim 11, wherein the sugar is glucose.

13. A chlorin derivative according to any one of claims 10 to 12, wherein the sugar is D-form.

14. A method of destroying a target in vitro or in vivo comprising:
contacting a chlorin derivative as claimed in any one of claims 1 to 13 with a target selected from the group consisting of virus, bacteria, or infected cell thereby, tumorous cell, and tumorous tissues; and
irradiating a light of wavelength absorbable to the chlorin derivative to destroy the target.

15. A medicine containing a compound as claimed in any one of claims 1 to 13 as an active ingredient.

16. A medicine for photodynamic therapy for a tumor or a solid cancer comprising a compound as claimed in any one of claims 1 to 13 as an active ingredient.

17. A medicine for photodynamic therapy for skin disease comprising a compound as claimed in any one of claims 1 to 13 as an active ingredient.

18. A pharmaceutical composition comprising a compound as claimed in any one of claims 1 to 13 as an active ingredient, and pharmaceutically acceptable diluents or carriers of solid or liquid.

19. Use of a compound as claimed in any one of claims 1 to 13 for medicine.

20. Use of a compound as claimed in any one of claims 1 to 13 for a medicine for treating diseases.

21. Use of a compound as claimed in any one of claims 1 to 13 for a diagnostic reagent.

22. A method of producing a chlorin derivative as claimed in any one of claims 1 to 3 comprising:
a step of preparing tetrakis(pentafluorophenyl)chlorin via a process of subjecting the A ring of tetraphenylporohyrin to a reduction reaction or an addition reaction to afford tetraphenylchrolin; and
a step of S-glycosylation by reacting the tetrakis(pentafluorophenyl)chlorin with a thiolester of a sugar or sugar derivative.

23. A method of producing a chlorin derivative as claimed in any one of claims 1 to 3 comprising:
a step of subjecting the A ring of tetrakis(pentafluorophenyl)porphyrin to a reduction reaction or an addition reaction to afford tetrakis(pentafluorophenyl)chrolin;
a step of separating and removing tetrakis(pentafluorophenyl)porphyrin from the resulting reaction mixture to obtain the tetrakis(pentafluorophenyl)chlorin; and
a step of S-glycosylation by reacting the tetrakis(pentafluorophenyl)chlorin with a thiolester of a sugar or sugar derivative.

24. A method of producing a chlorin derivative as claimed in any one of claims 4 to 8 comprising:
a step of preparing a metal complex of tetrakis(pentafluorophenyl)chlorin via a process of subjecting the A ring of tetraphenylporphyrin to a reduction reaction or an addition reaction to afford tetraphenylchrolin, and a process of reacting a tetraphenylporphyrin or tetraphenylchrolin with a salt of the metal; and
a step of S-glycosylation by reacting the metal complex of tetrakis(pentafluorophenyl)chlorin with a thiolester of a sugar or sugar derivative.

25. A method of producing a chlorin derivative as claimed in any one of claims 4 to 8 comprising:
a step of preparing a metal complex of tetrakis(pentafluorophenyl)porphyrin by reacting a tetrakis(pentafulorophenyl)porphyrin with a salt of the metal;
a step of subjecting the A ring of porphyrin of the metal complex of tetrakis(pentafluorophenyl)porphyrin to a reduction reaction or an addition reaction to afford a metal complex of tetrakis(pentafluorophenyl)chrolin;
a step of separating and removing the metal salt of tetrakis(pentafluorophenyl)porphyrin from the resulting reaction mixture to obtain the metal complex of tetrakis(pentafluorophenyl)chlorin; and
a step of S-glycosylation by reacting the metal complex of the tetrakis(pentafluorophenyl)chlorin with a thiolester of a sugar or sugar derivative.
